# EUROPEAN PATENT APPLICATION

(11) **EP 4 456 084 A1**
(43) Date of publication of application: **30.10.2024**
(21) Application number: 23170587.2
(22) Date of filing: 28.04.2023
(51) Int. Cl.: G16H 50/00, G16H 50/70

(54) **METHOD FOR DETERMINING A FUTURE HEALTH CONDITION OF AN INDIVIDUAL**

(71) Applicant: BULL SAS, 78340 Les Clayes-Sous-Bois (FR)
(72) Inventor: MITESH VIKRAM, KOTHARI, PIN 400080 Mumbai (IN)
(74) Representative: Cabinet Camus Lebkiri

(57) **Abstract**

A first aspect of the invention relates to a method for determining a future health condition of an individual. The method comprises:
- Providing health data of the individual,
- Obtaining a health condition information from the health data of the individual,
- Encoding the health data and the health condition information of the individual in a low-dimensional vector comprising the health data and the health condition information of the individual,
- Obtaining a trained neural network taking as input a low-dimensional vector of the individual and configured to determine a future health condition of the individual, and
- Determining a future health condition of the individual by inputting the health condition information of the individual to the trained neural network.

A second aspect of the invention relates to a system configured to carry out the method.

## Description

### TECHNICAL FIELD

The technical field of the invention is the one of machine learning and especially the one of machine learning for determining a future health condition of an individual.

The present invention regards a method for learning a neural network configured to determine a future health condition of an individual and a method for determining a future health condition of an individual. The present invention also regards a system configured to carry out the method.

### STATE OF THE ART

More and more health data are now digitalized. For example, these health data may be scans, doctor's prescriptions, blood sample reports, etc... These health data have various formats and are semi-structured and unstructured data. Semi-structured data is a type of data that doesn't respect the tabular structure associated with relational databases or other forms of data tables but contains tags and metadata to separate semantic elements and establish hierarchies of records and fields. Unstructured data is a type of data that is not organized according to a preset data model or schema, and therefore cannot be stored in a traditional relational database.

Health data are data comprising health condition information about individuals. Health condition information are meaningful information for a skilled person who is able to locate and understand these health condition information in the health data. A health condition information is an information about the health condition of an individual. It may be an information about the absence or a presence of a disease. It may also be an information about the probability of having or not having a disease in a predetermined time, like a day, a month or a year in the future. These health condition information could be used for various objectives such as determining or predicting a future health condition of an individual. However, due to the quantity and the variety of health data, it is a tremendous task to extract these health condition information from the health data.

Being able to determine a future health condition of an individual may be of great benefit. As an example, it is possible to change the lifestyle of the individual in order to prevent a disease to appear based on the determination of a future health condition of the individual. It could also help for evaluating needs of vaccines for an individual or any other prevention measures. It is also possible to use these information in various domains such as insurance. For example, it could help insurance companies to determine the right rider and premium terms based on the prediction. Indeed, after the COVID-19 crisis, insurer and insurance organization are facing lots of challenges to try to determine the right budget for health as insurer.

Machine learning methods are used for prediction. A prediction in machine learning refers to the output of a neural network that has been trained on a historical dataset. A neural network may be trained with an unsupervised learning. During the learning phase, an unsupervised neural network tries to mimic the data it's given and uses the error in its mimicked output to correct itself, by correcting its weights and biases. Sometimes the error is expressed as a low probability that the erroneous output occurs, or it might be expressed as an unstable high energy state in the network. One of the advantages of an unsupervised neural network is its ability to learn patterns from untagged data.

There is therefore a need to provide a method which is able to determine a future health condition of an individual by leveraging the large existing semi structured and unstructured health data.

### SUMMARY OF THE INVENTION

According to a first aspect of the invention, this need is satisfied by providing a computer implemented method of machine learning comprising:
- Providing, for a set of individuals, unstructured and/or semi structured health data comprising at least one amongst:
   - family history health,
   - genetic information,
   - individual nature,
   - body structure,
   - face structure,
   - pathology reports,
- Obtaining, for each individual of the set of individuals, a health condition information from the health data,
- Generating a first graph based on the health data and the health condition information, each node of the first graph comprising the health data and the health condition information of one individual of the set of individuals and being connected to at least one other node of the first graph,
- Applying a node embedding algorithm to the first graph, the node embedding algorithm comprising an encoding of each node of the first graph as a low-dimensional vector comprising the health data and the health condition information of the node, a position of the node in the first graph and a structure of the node's local first graph neighbourhood, therefore obtaining a first set of low-dimensional vectors, and
- Learning a neural network taking as input a low-dimensional vector and configured to determine a future health condition of the individual, the learning being performed with an unsupervised training based on the first set of low-dimensional vectors and comprising applying a similarity algorithm to identify, for each vector of the first set of low-dimensional vectors, similar vectors in the first set of low-dimensional vectors.

In addition to the characteristics which have just been mentioned in the previous paragraph, the method according to one aspect of the invention may have one or more additional characteristics among the following, considered individually or according to all technically possible combinations:
- The obtaining a health condition information comprises:
   - Extracting the health condition information from at least one of the unstructured and/or semi structured health data using optical character recognition and/or intelligent character recognition and/or natural language processing technologies, and/or
   - Accessing and retrieving the health condition information from healthcare organization database based on historical data or records, and/or
   - Receiving the health condition information from a user using a single page application considering recent activities and medical history in question-and-answer form.
- The generating a first graph comprises:
   - Converting the health data in a tree data structure, the tree data structure having a root node corresponding to one individual of the set of individuals and at least one leaf substructure, each leaf substructure comprising one type of health data, and/or
   - Transforming the converted health data into a graph database format.
- The method also comprises:
   - Obtaining at least one medical knowledge repository,
   - Extracting medical knowledges from at least a one of the medical knowledge repository using optical character recognition and/or intelligent character recognition and/or natural language processing technologies,
   - Generating a second graph based on the medical knowledges, each node of the second graph comprising one medical knowledge of the extracted medical knowledges and being connected to at least one other node of the second graph,
   - Applying a node embedding algorithm to the second graph, the node embedding algorithm comprising an encoding of each node of the second graph as a low-dimensional vector comprising the medical knowledge of the node, a position of the node in the second graph and a structure of the node's local second graph neighbourhood, therefore obtaining a second set of low-dimensional vectors,
   wherein during the learning of the neural network, the second set of low-dimensional vectors is used to identify, for each vector of the first set of low-dimensional vectors, similar vectors in the first set of low-dimensional vectors.
- additional unstructured and/or semi structured health data are obtained and used to update, at a predefined interval of time, the learned neural network.

A second aspect of the invention relates to a method for determining a future health condition of an individual comprising:
- Providing health data of the individual,
- Obtaining a health condition information from the health data of the individual,
- Encoding the health data and the health condition information of the individual in a low-dimensional vector comprising the health data and the health condition information of the individual,
- Obtaining a trained neural network taking as input a low-dimensional vector of the individual and configured to determine a future health condition of the individual, and
- Determining a future health condition of the individual by inputting the health condition information of the individual to the trained neural network.

In addition to the characteristics which have just been mentioned in the previous paragraph, the method according to one aspect of the invention may have one or more additional characteristics among the following, considered individually or according to all technically possible combinations:
- The method further comprises measuring at least one vital sign,
- The method further comprises generating and outputting a recommendation on preventing a future health condition of an individual,
- The method further comprises identifying and outputting an emergency alert,
- The method further comprises generating a recommended rider and/or a premium term for healthcare insurance organization based on the future health condition of the individual.

A third aspect of the invention relates to a computer program comprising instructions which, when the computer program is carried out on a computer, cause the computer to carry out the method according to the invention.

A fourth aspect of the invention relates to a system comprising a processor coupled to a memory, the memory having recorded thereon the computer program of the invention.

In addition to the characteristics which have just been mentioned in the previous paragraph, the system according to one aspect of the invention may have one or more additional characteristics among the following, considered individually or according to all technically possible combinations:
- The system is configured to measure at least one vital sign,
- The system is configured to output a recommendation on preventing a future health condition of an individual and/or an emergency alert.

### BRIEF DESCRIPTION OF THE FIGURES

Other characteristics and advantages of the invention will become clear from the description that is given thereof below, by way of indication and in no way limiting, with reference to the appended figures, among which:
Figure 1 shows a flowchart of an example of the computer-implemented method of machine learning according to the invention.
Figures 2 and 3 show a flowchart of an example of a step of the computer-implemented method of machine learning according to the invention.
Figure 4 shows a flowchart of an example of the computer-implemented method for determining a future health condition of an individual according to the invention.
Figure 5 shows an example of a tree data structure comprising health data according to the invention.

### DETAILED DESCRIPTION

For greater clarity, identical or similar elements are marked by identical reference signs in all of the figures.

Some embodiments of devices and methods in accordance with embodiments of the present invention are now described, by way of example only, and with reference to the accompanying drawings. The description is to be regarded as illustrative in nature and not as restrictive.

A first aspect of the invention concerns a method of learning a neural network configured to determine a future health condition of the individual.

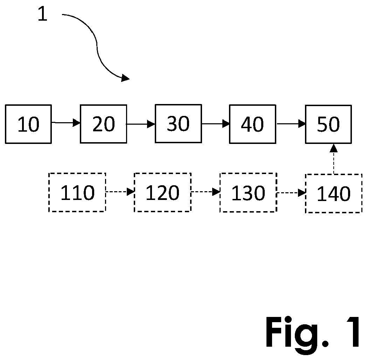

Figure 1 is a synoptic scheme illustrating the steps of an example of the method 1 according to the invention. The mandatory steps of the example of the method 1 are indicated by a rectangle with solid lines and the optional steps are indicated by a rectangle with dashed lines.

A first step 10 consists of providing, for a set of individuals, unstructured and/or semi structured health data. The set of individuals may comprise between 10000 and 100000 individuals. The set of individuals may be representative of a specific population such as a population of a class of age or a population of a country. The set of individuals may also be a set of random individuals. The health data comprise various data. For example, the health data comprise family history health and genetic information. The health data also comprise individual nature. Individual nature of an individual may be one among the following predetermined categories: honest, shrewd, artistic, sympathy, irritable, etc... The health data comprise also body and face structure and pathology reports. Pathology reports may have information for an individual, regarding their level of vitamins, weight, CBC for "complete blood count", lipid profile, sugar, uric acid, creatine. A CBC is a blood test. It provides an insight of an overall health and find a wide range of conditions, including anemia, infection and leukemia. A complete blood count test measures the following:
- Red blood cells, which carry oxygen,
- White blood cells, which fight infection,
- Hemoglobin, the oxygen-carrying protein in red blood cells,
- Hematocrit, the amount of red blood cells in the blood,
- Platelets, which help blood to clot.

The health data may also comprise data regarding demographic details, intelligent and emotional quotient, diet patterns, existing and/or past diseases, desires and aversions, body reactions to temperature and/or weather, body temperature, nature and/or pet affinity. Some data such as family history, body thermal, pathology reports, diet patterns, may be text. Other data such as body and face structure may be photos or pictures. The term "providing" means "receiving, accessing or computing by the computer".

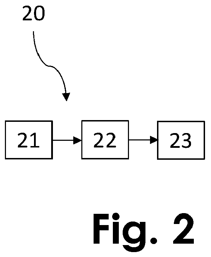

A second step 20 consists of obtaining health condition information from the health data. Figure 2 shows a flowchart of an example of the step 20. As an example, the step of obtaining 20 may comprise a sub step 21 of extracting a health condition information. The extraction may be performed by optical character recognition and/or intelligent character recognition and/or natural language processing technologies. In another example, compatible with the precedent example, the obtaining 20 may comprise a sub step 22 of accessing and retrieving the health condition information from healthcare organization database based on historical data or records. In another example, compatible with the precedent examples, the obtaining 20 may comprise a sub step 23 of receiving the health condition information from a user using a single page application considering recent activities and medical history in question-and-answer form.

A third step 30 consists of generating a first graph. A graph is a structure made of nodes and edges between the nodes. The first graph is generated by using the health data and the health condition information. Each node of the first graph comprising the health data and the health condition information of one individual of the set of individuals. Therefore, each node corresponds to one individual of the set of individuals. The health data and the health condition information of one node may be stored in a data structure. Each node is connected by one or more edges to at least one other node of the first graph. An edge may be added between two nodes if the health data and the health condition information of two nodes are similar. By "similar", it is meant for example that 80% of the health data and the health condition information are identical, i.e., of same values within a predetermined range, between the two nodes.

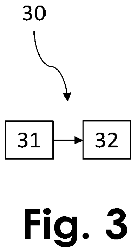

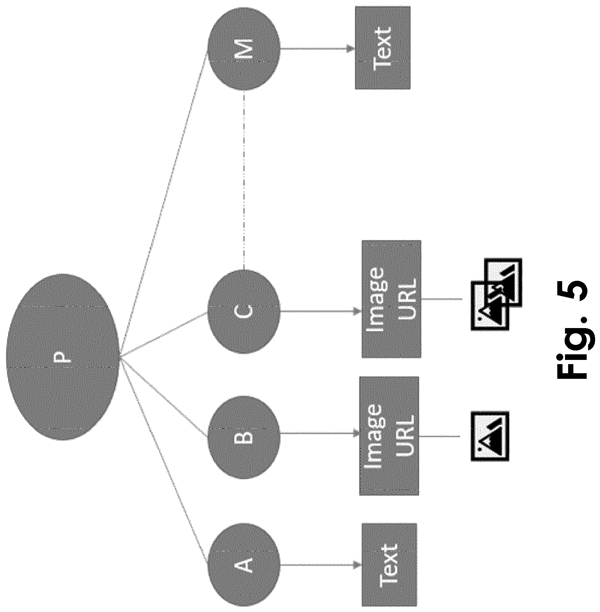

As an example, the third step 30 may comprise two sub steps. Figure 3 shows a flowchart of an example of the step 30. The first sub step 31 of the step 30 may consist of converting the health data in a tree data structure, the tree data structure having a root node corresponding to one individual of the set of individuals and at least one leaf sub structure. Each leaf substructure comprising one type of data. For example, as illustrated in figure 5, the root node, noted P, has children leaf substructures noted A, B, C, ..., N. As an example, a tree data structure may have the following structure or part of the following structure:
Person Construct {
A. Demographic details: containing text information;
B. Face structure (eye, nose, ears): containing Image Photo reference (URL);
C. Physical structure of body: Image: containing Photo of overall structure URL);
D. Nature (honest, shrewd, artistic, sympathy, gets hurt easily): containing Text information;
E. IQ & EO: capture the information with specific queries;
F. Pathology reports: containing information about Vitamins, weight, CBC, lipid profile, sugar, uric acid, creatine (ICR/OCR);
G. Desire and aversion (like, dislike, salty food): containing text information;
H. Food pattern: containing text information;
I. Any existing disease: containing text information;
J. Body reaction (As per weather, temp): containing text information;
K. Body thermal (hot, cold): containing text information;
L. Family history: containing text information;
M. Nature lover, animal lover: containing text information;
N. Existing Diseases: containing text information;}

As an example, all or a part of these types of data may be health data provided at step 10. Furthermore, the more health data is provided, the more the neural network configured to determine a future health condition of an individual is accurate. By "the more data is provided", it is meant, more variety of types of health data and/or greater number of records of each type of health data.

As an example, the duplicated health data may also be removed during the sub step 31. The sub step 31 may also comprise creating reference links for each unstructured health data to the relational data in table and/or converting each table data value of captured system into intermediate format.

A second sub step 32 of the step 30 may consist of transforming the converted health data into a graph database format.

A fourth step 40 consists of applying a node embedding algorithm to the first graph. The node embedding algorithm comprises an encoding of the nodes of the first graph. The encoding encodes each node as a low-dimensional vector. The low-dimensional vector refers here to vector of a dimension between 800 and 80000 dimensions. The low-dimensional vector comprises the health data and the health condition information of the node, a position of the node in the first graph and a structure of the node's local first graph neighbourhood. By structure of the node's local first graph neighbourhood, it is meant that the low-dimensional vector comprises data about the one or more connections of the node. By applying at the step 40 a node embedding algorithm to the first graph, a first set of low-dimensional vectors is obtained.

A fifth step 50 consists of learning a neural network configured to determine a future health condition of the individual. The neural network takes as input the low-dimensional vector. The learning 50 is performed with an unsupervised training. The learning is performed by using the first set of low-dimensional vectors as training data. The learning comprises applying a similarity algorithm to identify, for each vector of the first set of low-dimensional vectors, similar vectors in the first set of low-dimensional vectors.

In an example, compatible with the precedent examples, the method 1 also uses medical knowledge for learning a neural network configured to determine a future health condition of the individual. In this example, four additional steps are performed before the step of learning 50 the neural network. The first additional step 110 consists of obtaining one or more medical knowledge repositories. A medical knowledge repository is a repository containing medical knowledge such as the symptoms of a disease and/or the treatment of such disease. A second additional step 120 consists of extracting medical knowledges from the medical knowledge repositories. The extraction may be performed by using optical character recognition and/or intelligent character recognition and/or natural language processing technologies. A third additional step 130 consists of generating a second graph. The second graph is built based on the medical knowledges. Each node of the second graph comprises one medical knowledge of the extracted medical knowledges. For example, each node of the second graph comprises a medical knowledge about one disease. Each node of the second graph is connected to at least one other node of the second graph. An edge between two nodes of the second graph is added based on the similarity between the medical knowledges of the two nodes. For example, if 80% of the symptoms of the medical knowledge of two nodes are similar, an edge may be added between these two nodes. In another example, if 80% of the data of two nodes are similar, an edge may be added between these two nodes. A fourth additional step 140 consists of applying a node embedding algorithm to the second graph. The node embedding algorithm applied to the second graph may be the same than the node embedding algorithm applied to the first graph. Each low-dimensional vector comprises the medical knowledge of the node, a position of the node in the second graph and a structure of the node's local second graph neighbourhood. By applying at the step 140 a node embedding algorithm to the second graph, a second set of low-dimensional vectors is obtained. This second set of low-dimensional vectors is used during the learning 50 of the neural network. More specifically, the second set of low-dimensional vectors is used to identify, for each vector of the first set of low-dimensional vectors, similar vectors in the first set of low-dimensional vectors. By using the second set of low-dimensional vectors, the learning is more efficient to identify, the similar vectors of the first set of low-dimensional vectors. Therefore, the neural network obtained is more precise for determining a future health condition of an individual.

In an example, compatible with the precedent examples, the learned neural network may be updated with additional unstructured and/or semi structured health data. To update the neural network, the steps 10 to 50 may be performed with the additional unstructured and/or semi structured health data.

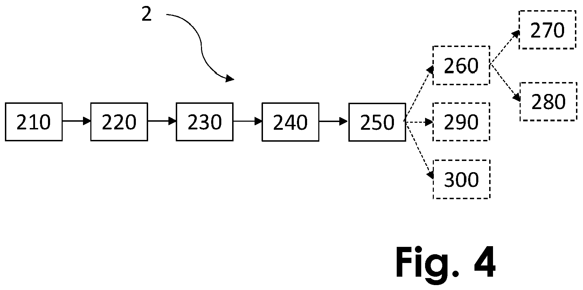

A second aspect of the invention concerns a method 2 for determining a future health condition of an individual. Figure 4 is a synoptic scheme illustrating the steps of an example method 2 according to the invention. The mandatory steps of the example method 2 are indicated by a rectangle with solid lines and the optional steps are indicated by a rectangle with dashed lines.

A first step 210 consists of providing health data of the individual. These health data may be of any type and may comprise family history health and/or genetic information and/or individual nature and/or body and face structure and/or pathology reports.

A second step 220 consists of obtaining a health condition information from the health data of the individual. The step 220 may be performed in the same manner than the step 20.

A third step 230 consists of encoding the health data of the individual and the health condition information in a low-dimensional vector. The low-dimensional vector comprises the health data and the health condition information of the individual.

A fourth step 240 consists of obtaining a trained neural network taking as input the low-dimensional vector of the individual and configured to determine a future health condition of the individual. The neural network may have been learned by using the method 1 of machine learning of the invention. In this case, the individual whom health data have been provided is not comprised in the set of individuals used for the learning of the neural network.

A fifth step 250 consists of determining a future health condition of the individual by inputting the health condition information of the individual to the trained neural network.

In an example, a sixth additional step 260 may consist of measuring at least one vital sign. By "measuring", it is meant detecting or acquiring or receiving data for at least one vital sign. For example, the one or more vital signs detected may be the heart rate and/or respiratory rate and/or body temperature and/or oxygen saturation and/or blood pressure of the individual.

In an example, compatible with the precedent example, a seventh optional step 270 may consist of generating and outputting a recommendation on preventing a future health condition of an individual. By "outputting", it is meant displaying, storing or sending data to another device. The recommendation may consist for example of changing the lifestyle and/or the food diet of the individual.

In an example, compatible with the precedent examples, an eighth optional step 280 may consist of identifying and outputting an emergency alert. The identifying may consist of detecting an anomaly of a vital sign and determining if an emergency alert must be send based on the anomaly and the health data, the health condition information and the future health condition of the individual determined by the trained neural network.

In an example, a ninth optional step 290 may consist of generating. a recommended rider and/or a premium term for healthcare insurance organization based on the future health condition of the individual based on the future health condition of the individual determined by the trained neural network. The recommended riders and/or a premium term may also be updated and/or modified based on user inputs.

In an example, a tenth optional step 300 may consist of generating recommendations about optional or mandatory vaccines in order to reduce the risk of an individual of getting a disease. The recommendations are based on the determined future health condition of the individual.

The methods 1 and 2 are "computer-implemented". "Computer-implemented" means that the steps, or at least some of the steps, are performed by at least one computer or processor or other similar system. Thus, steps are performed by the computer, possibly fully automatically, or, semi-automatically. In examples, the triggering of at least some of the steps of these methods may be performed by user-computer interaction. The level of user-computer interaction required may depend on the level of automation intended and balanced against the need to implement the user's wishes. In examples, this level may be user defined and/or may be predefined.

A typical example of a computer implementation of a method is to execute the method with a system adapted for the purpose. A system may be configured to implement all the steps of the method and/or its various embodiments according to the invention. To this end, the system comprises a memory and a computing unit, the memory being configured to store instructions which, when executed by the computing unit, cause the computing unit to implement the steps of the method according to the invention and/or the different embodiments of the method according to the invention. The system further comprises at least one network interface for communicating with remote entities, i.e., for sending and receiving data to said entities, via at least one network. The entities may be servers or computers storing data. The data may be stored by the same server or by different servers/databases.

In an example, the system may be configured to measure at least one vital sign. For example, it may do so using a smart watch or a wearable fitness tracker or a blood pressure monitor it comprises, or if the system in itself is such a wearable, etc...

In another example, compatible with the precedent example, the system may be configured to output a recommendation on preventing a future health condition of an individual and/or an emergency alert. For example, the system may have a screen to display the recommendation or the emergency alert. It may also send the recommendation or the alert to another device, such as a smartphone or another computer.

The computer program may comprise instructions executable by a computer, the instructions comprising means for causing the above system to perform the methods. The program may be recordable on any data storage medium, including the memory of the system. The program may for example be implemented in digital electronic circuitry, or in computer hardware, firmware, software, or in combinations of them. The program may be implemented as an apparatus, for example a product tangibly embodied in a machine-readable storage device for execution by a programmable processor. Method steps may be performed by a programmable processor executing a program of instructions to perform functions of the methods by operating on input data and generating output. The processor may thus be programmable and coupled to receive data and instructions from, and to transmit data and instructions to, a data storage system, at least one input device, and at least one output device. The application program may be implemented in a high-level procedural or object-oriented programming language, or in assembly or machine language if desired. In any case, the language may be a compiled or interpreted language. The program may be a full installation program or an update program. Application of the program on the system results in any case in instructions for performing the methods.

## Claims

1. A computer implemented method (1) of machine learning comprising:
- Providing (10), for a set of individuals, unstructured and/or semi structured health data comprising at least one amongst:
∘ family history health,
∘ genetic information,
∘ individual nature,
∘ body structure,
∘ face structure,
∘ pathology reports,
- Obtaining (20), for each individual of the set of individuals, health condition information from the health data,
- Generating (30) a first graph based on the health data and the health condition information, each node of the first graph comprising the health data and the health condition information of one individual of the set of individuals and being connected to at least one other node of the first graph,
- Applying (40) a node embedding algorithm to the first graph, the node embedding algorithm comprising an encoding of each node of the first graph as a low-dimensional vector comprising the health data and the health condition information of the node, a position of the node in the first graph and a structure of the node's local first graph neighbourhood, therefore obtaining a first set of low-dimensional vectors, and
- Learning (50) a neural network taking as input a low-dimensional vector and configured to determine a future health condition of the individual, the learning being performed with an unsupervised training based on the first set of low-dimensional vectors and comprising applying a similarity algorithm to identify, for each vector of the first set of low-dimensional vectors, similar vectors in the first set of low-dimensional vectors.

2. A computer implemented method (1) of claim 1 wherein the obtaining (20) a health condition information comprises:
- Extracting (21) the health condition information from at least one of the unstructured and/or semi structured health data using optical character recognition and/or intelligent character recognition and/or natural language processing technologies, and/or
- Accessing (22) and retrieving the health condition information from healthcare organization database based on historical data or records, and/or
- Receiving (23) the health condition information from a user using a single page application considering recent activities and medical history in question-and-answer form.

3. A computer implemented method (1) of claim 1 or 2 wherein the generating (30) a first graph comprises:
- Converting (31) the health data in a tree data structure, the tree data structure having a root node corresponding to one individual of the set of individuals and at least one leaf substructure (A, B, C,..., M), each leaf substructure comprising one type of health data, and/or
- Transforming (32) the converted health data into a graph database format.

4. A computer implemented method (1) of any of the precedent claims also comprising:
- Obtaining (110) at least one medical knowledge repository,
- Extracting (120) medical knowledges from at least a one of the medical knowledge repository using optical character recognition and/or intelligent character recognition and/or natural language processing technologies,
- Generating (130) a second graph based on the medical knowledges, each node of the second graph comprising one medical knowledge of the extracted medical knowledges and being connected to at least one other node of the second graph,
- Applying (140) a node embedding algorithm to the second graph, the node embedding algorithm comprising an encoding of each node of the second graph as a low-dimensional vector comprising the medical knowledge of the node, a position of the node in the second graph and a structure of the node's local second graph neighbourhood, therefore obtaining a second set of low-dimensional vectors,
wherein during the learning (50) of the neural network, the second set of low-dimensional vectors is used to identify, for each vector of the first set of low-dimensional vectors, similar vectors in the first set of low-dimensional vectors.

5. A computer implemented method (1) of any of the precedent claims wherein additional unstructured and/or semi structured health data are obtained and used to update, at a predefined interval of time, the learned neural network.

6. A computer implemented method (2) for determining a future health condition of an individual:
- Providing (210) health data of the individual,
- Obtaining (220) a health condition information from the health data of the individual,
- Encoding (230) the health data and the health condition information of the individual in a low-dimensional vector comprising the health data and the health condition information of the individual,
- Obtaining (240) a trained neural network taking as input a low-dimensional vector of the individual and configured to determine a future health condition of the individual, and
- Determining (250) a future health condition of the individual by inputting the health condition information of the individual to the trained neural network.

7. A computer implemented method (2) of claim 6 further comprising measuring (260) at least one vital sign.

8. A computer implemented method of claim 6 or 7 further comprising generating and outputting (270) a recommendation on preventing a future health condition of an individual.

9. A computer implemented method of claims 6 to 8 further comprising identifying and outputting (280) an emergency alert.

10. A computer implemented method of claim 6 further comprising generating (290) a recommended rider and/or a premium term for healthcare insurance organization based on the future health condition of the individual.

11. A computer program comprising instructions which, when the computer program is carried out on a computer, cause the computer to carry out the method (1) according of any one of claims 1 to 5 or the method (2) according of any one of claims 6 to 10.

12. A computer-readable data storage medium having recorded thereon the computer program of claim 11.

13. A system comprising a processor coupled to a memory, the memory having recorded thereon the computer program of claim 11.

14. A system of claim 11 configured to measure at least one vital sign.

15. A system of claim 11 configured to output a recommendation on preventing a future health condition of an individual and/or an emergency alert.
